# EUROPÄISCHE PATENTSCHRIFT

(11) **EP 1 110 942 B1**
(45) Veröffentlichungstag und Bekanntmachung des Hinweises auf die Patenterteilung: **01.10.2003**
(21) Anmeldenummer: 00115959.9
(22) Anmeldetag: 26.07.2000
(51) Int. Cl.: C07C 211/51, A61K 7/13, C07C 209/68, C07C 209/04, C07C 209/30

(54) **Verwendung von 2-Aminomethyl-1,4-diamino-benzol und dessen Salzen in Färbemitteln für Keratinfasern**
Use of 2-aminomethyl-1,4-diamino-benzene and salts thereof in dyeing composition for keratin fibers
L'utilisation du 2-aminométhyl-1,4-diamino-benzène et ses sels dans des compositions de teinture des fibres kératiniques

(30) Priorität: 18.12.1999 DE 19961229
(43) Veröffentlichungstag der Anmeldung: 27.06.2001
(60) Teilanmeldung: 03003356.7
(73) Patentinhaber: Wella Aktiengesellschaft, 64295 Darmstadt (DE)
(72) Erfinder: Göttel, Otto, Dr., 1723 Marly (CH); Pirrello, Aline, 1762 Givisiez (CH); Hayoz, André, 1724 Senèdes (CH)

(56) Entgegenhaltungen:
- WO-A-98/01106
- HANS-JOACHIM KABBE: "Heterocyclen aus Diaminen und Diacylverbindungen" JUSTUS LIEBIGS ANNALEN DER CHEMIE, VERLAG CHEMIE GMBH. WEINHEIM, DE, 1978, Seiten 398-404, XP002184522

## Beschreibung

Gegenstand der vorliegenden Anmeldung ist die Verwendung von 2-Aminomethyl-1,4-diamino-2- benzol oder dessen physiologisch verträglichen Salzen mit organischen oder anorganischen Säuren als Farbstoff-Vorstufe in Färbemitteln für Keratinfasern sowie 2-Aminomethyl-1,4-diamino-2- benzol oder dessen Salze enthaltende Oxidationsfärbemittel für Keratinfasern.

Das 2-Aminomethyl-1,4-diamino-2- benzol ist aus Liebigs Annalen der Chemie 1978, Seite 398-404 als Ausgangsverbindung für die Herstellung von bestimmten Heterozyklen bekannt. Die Verwendung dieser Verbindung in Färbemitteln für Keratinfasern wird dort jedoch nicht beschrieben.
Weiterhin sind aus der WO 98/01106 Oxidationsfärbemittel bekannt, welche als Farbstoffvorstufe ein 2-Amino-1,4-diaminobenzol-derivat enthalten.

Gegenstand der vorliegenden Erfindung ist daher die Verwendung 2-Aminomethyl-1,4-diamino-2- benzol oder dessen Salzen der Formel (I) in Färbemitteln für Keratinfasern, beispielsweise Wolle, Seide oder Haaren, insbesondere menschlichen Haaren, 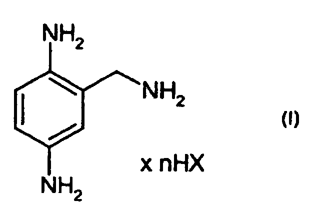 worin n einen Wert von 0 bis 3 aufweist und HX für eine organische oder anorganische Säure steht. Als Säuren können anorganische oder organische Säuren eingesetzt werden, wobei die folgenden Säuren bevorzugt sind: Salzsäure, Schwefelsäure, Borsäure, Zitronensäure und Weinsäure. Besonders bevorzugt sind Salzsäure und Schwefelsäure.

Infolge der hohen Oxidationsempfindlichkeit der freien Base (Formel (I), n=0) wird die Verbindung der Formel (I) bevorzugt als Säureaddukt (n≠0) eingesetzt.

Obwohl sich die Verbindungen der Formel (I) insbesondere für die Verwendung zur Färbung von Keratinfasern eignen, ist es prinzipiell auch möglich mit diesen Verbindungen andere natürliche oder synthetische Fasern, beispielsweise Baumwolle oder Nylon 66, zu färben.

Die Verbindungen der Formel (I) eignen sich hervorragend als Farbstoff-Vorstufen im oxidativen System zum Färben von Keratinfasern, wobei die Verbindungen der Formel (I) sowohl alleine als auch in Kombination mit bestimmten bekannten Entwicklersubstanzen und/oder Kupplersubstanzen, die in oxidativen Färbesystemen zur Färbung von Fasermaterialien üblicherweise verwendet werden, eingesetzt werden können.

Als geeignete Kupplersubstanzen können insbesondere genannt werden: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methyl-benzol, 2,4-Diamin-1-ethoxy-5-methylbenzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxypyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1-(3-hydroxypropoxy)-benzol, 1-(2-Aminoethoxy)-2,4-diamino-benzol, 2-Amino-1-(2-hydroxyethoxy)-4-methylamino-benzol, 2,4-Diaminophenoxyessigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)-amino]-acetamid, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)-amino]-2-methyl-phenol, 3-[(2-Hydroxyethyl)amino]-2-methyl-phenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxyphenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion, oder deren Salze.

Zur Herstellung insbesondere von Naturtönen und modischen Rottönen ist es besonders vorteilhaft, Verbindungen der Formel (I) in Kombination mit zusätzlichen Entwicklersubstanzen einzusetzen. Als Entwicklersubstanzen kommen Paraphenylendiamine, Paraaminophenole sowie 4,5-Diaminopyrazole oder deren Salze in Betracht. Insbesondere sind die folgenden Entwicklersubstanzen zu nennen:
1,4-Diamino-benzol (p-Phenylendiamin), 1,4-Diamino-2-methyl-benzol (p-Toluylen-diamin), 1,4-Diamino-2,6-dimethyl-benzol, 1,4-Diamino-2,5-dimethyl-benzol, 1,4-Diamino-2,3-dimethyl-benzol, 2-Chlor-1,4-diaminobenzol, 4-Phenylamino-anilin, 4-Dimethylamino-anilin, 4-Diethylaminoanilin, 4-[Di(2-hydroxyethyl)amino]-anilin, 4-[(2-Methoxyethyl)amino]-anilin, 4-[(3-Hydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)-(2-hydroxyethyl)amino]-2-propanol, 1,8-Bis(2,5-diaminophenoxy)-3,6-dioxaoctan, 4-Amino-phenol, 4-Amino-3-methyl-phenol, 4-Methylaminophenol, 4-Amino-2-(amminomethyl)-phenol, 4-Amino-2-[(2-hydroxyethyl)-amino]methyl-phenol, 4-Amino-2-(methoxy-methyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1H)-pyrimidon, 4,5-Diamino-1-(2-hydroxyethyl)-1H-pyrazol, 4,5-Diamin-1-(1-methylethyl)-1H-pyrazol, 4,5-Diamino-1-[(4-methyl-phenyl)methyl]-1H-pyrazol, 1-[(4-Chlorphenyl)methyl]-4,5-diamino-1H-pyrazol, 4,5-Diamino-1-methyl-1H-pyrazol, 4,5-Diamino-3-methyl-1-phenyl-1H-pyrazol, 4,5-Diamino-1-(2-hydroxyethyl)-3-methyl-1H-pyrazol, 2-Amino-phenol, 2-Amino-6-methylphenol und 2-Amino-5-methyl-phenol, oder deren Salze.

Die Verbindungen der Formel (I) können selbstverständlich auch in Kombination mit üblichen direktziehenden anionischen, kationischen oder neutralen Farbstoffe verwendet werden. Zu den bevorzugten anionischen Farbstoffen zählen beispielsweise 6-Hydroxy-5-[(4-sulfophenyl)azo]-2-naphthalinsulfonsäure-dinatriumsalz (CI15985; Food Yellow No. 3; FD&C Yellow No. 6), 2,4-Dinitro-1-naphthol-7-sulfonsäure-dinatriumsalz (CI10316; Acid Yellow No. 1; Food Yellow No. 1), 2-(Indan-1,3-dion-2-yl)chinolin-x,x-sulfonsäure (Gemisch aus Mono- und Disulfonsäure) (CI47005;D&C Yellow No. 10; Food Yellow No. 13, Acid Yellow No. 3), 5-Hydroxy-1-(4-sulfophenyl)-4-[(4-sulfophenyl)azo]pyrazol-3-carbonsäuretrinatriumsalz (CI19140; Food Yellow No. 4; Acid Yellow No. 23), 9-(2-Carboxyphenyl)-6-hydroxy-3H-xanthen-3-on (CI45350; Acid Yellow No. 73; D&C Yellow No. 8), 5-[(2,4-Dinitrophenyl)amino]-2-phenylaminobenzolsulfonsäure-natriumsalz (CI10385; Acid Orange No. 3), 4-[(2,4-Dihydroxyphenyl)azo]-benzolsulfonsäure-mononatriumsalz (CI14270; Acid Orange No. 6), 4-[(2-Hydroxynaphth-1-yl)azo]-benzolsulfonsäurenatriumsalz (CI15510; Acid Orange No. 7), 4-[(2,4-Dihydroxy-3-[(2,4-dimethylphenyl)azo]phenyl)azo]-benzolsulfonsäure-natriumsalz (CI20170; Acid Orange No. 24), 4-Hydroxy-3-[(4-sulfonaphth-1-yl)azo]-1-naphthalinsulfonsäure-dinatriumsalz (CI14720; Acid Red No. 14), 6-Hydroxy-5-[(4-sulfonaphth-1-yl)azo]-2,4-naphthalin-disulfonsäure-trinatriumsalz (CI16255; Ponceau 4R; Acid Red No. 18), 3-Hydroxy-4-[(4-sulfonaphth-1-yl)azo]-2,7-naphthalin-disulfonsäure-trinatriumsalz (CI16185; Acid Red No. 27), 8-Amino-1-hydroxy-2-(phenylazo)-3,6-naphthalin-disulfonsäuredinatriumsalz (CI17200; Acid Red No. 33), 5-(Acetylamino)-4-hydroxy-3-[(2-methylphenyl)azo]-2,7-naphthalin-disulfonsäure-dinatriumsalz (CI18065; Acid Red No. 35), 2-(3-Hydroxy-2,4,5,7-tetraiod-dibenzopyran-6-on-9-yl)-benzoesäure-dinatriumsalz (CI45430;Acid Red No. 51), N-[6-(Diethylamino)-9-(2,4-disulfophenyl)-3H-xanthen-3-yliden]-N-ethylethanaminium-hydroxid, inneres Salz, Natriumsalz (CI45100; Acid Red No. 52), 8-[(4-(Phenylazo)phenyl)azo]-7-naphthol-1,3-disulfonsäuredinatriumsalz (CI27290; Acid Red No. 73), 2',4',5',7'-Tetrabrom-3',6'dihydroxyspiro[isobenzofuran-1(3H),9'-[9H]xanthen]-3-on-dinatriumsalz (CI45380; Acid Red No. 87), 2',4',5',7'-Tetrabrom-4,5,6,7-tetrachlor-3',6'dihydroxyspiro[isobenzofuran-1(3H),9'[9H]xanthen]-3-on-dinatriumsalz (CI45410; Acid Red No. 92), 3',6'-Dihydroxy-4',5'-diiodospiro[isobenzofuran-1 (3H),9'(9H)-xanthen)-3-on-dinatriumsalz (CI45425; Acid Red No. 95), (2-Sulfophenyl)di[4-(ethyl((4-sulfophenyl)methyl)amino)-phenyl]-carbenium-dinatriumsalz, betain (CI42090; Acid Blue No. 9; FD&C Blue No. 1), 1,4-Bis[(2-sulfo-4-methylphenyl)amino]-9,10-anthrachinondinatriumsalz (CI 61570; Acid Green No. 25), Bis[4-(dimethylamino)-phenyl]-(3,7-disulfo-2-hydroxynaphth-1-yl)carbenium-inneres Salz, mononatriumsalz (CI44090; Food Green No. 4; Acid Green No. 50), Bis[4-(diethylamino)phenyl](2,4-disulfophenyl)carbenium-inneres salz, Natriumsalz (2:1) (CI42045; Food Blue No. 3; Acid Blue No. 1), Bis[4-(diethylamino)phenyl](5-hydroxy-2,4-disulfophenyl)carbeniuminneres salz, Calciumsalz (2:1) (CI42051; Acid Blue No. 3), 1-Amino-4-(cyclohexylamino)-9,10-anthrachinon-2-sulfonsäure-natriumsalz (CI62045; Acid Blue No. 62), 2-(1,3-Dihydro-3-oxo-5-sulfo-2H-indol-2-yliden)-2,3-dihydro-3-oxo-1H-indol-5-sulfonsäure-dinatriumsalz (CI73015; Acid Blue No. 74), 9-(2-Carboxyphenyl)-3-[(2-methylphenyl)amino]-6-[(2-methyl-4-sulfophenyl)amino]xanthylium-inneres Salz, mononatriumsalz (CI45190; Acid Violet No. 9), 1-Hydroxy-4-[(4-methyl-2-sulfophenyl)amino]-9,10-anthrachinon-natriumsalz (CI60730; D&C Violett No. 2; Acid Violet No. 43), Bis[3-nitro-4-[(4-phenylamino)-3-sulfo-phenylamino]-phenyl]-sulfon (CI10410; Acid Brown No. 13), 5-Amino-4-hydroxy-6-[(4-nitrophenyl)azo]-3-(phenylazo)-2,7-naphthalin-disulfonsäure-dinatriumsalz (CI20470; Acid Black No. 1), 3-Hydroxy-4-[(2-hydroxynaphth-1-yl)azo]-7-nitro-1-naphthalin-sulfonsäure-chromkomplex (3:2) (CI15711; Acid Black No. 52), 3-[(2,4-Dimethyl-5-sulfophenyl)azo]-4-hydroxy-1-naphthalin-sulfonsäuredinatriumsalz (CI14700; Food Red No. 1; Ponceau SX; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-tetranatriumsalz (CI28440; Food Black No. 1), 3-Hydroxy-4-(3-methyl-5-oxo-1-phenyl-4,5-dihydro-1H-pyrazol-4-ylazo)-naphthalin-1-sulfonsäure natriumsalz Chrom-Komplex (Acid Red No. 195).

Zu den bevorzugten kationischen Farbstoffen zählen beispielsweise 9-(Dimethylamino)-benzo[a]phenoxazin-7-ium-chlorid (CI51175; Basic Blue No. 6), Di[4-(diethylamino)phenyl][4-(ethylamino)naphthyl]carbeniumchlorid (CI42595; Basic Blue No. 7), 3,7-Di(dimethylamino)phenothiazin-5-ium-chlorid (CI52015; Basic Blue No. 9), Di[4-(dimethylamino)phenyl][4-(phenylamino)naphthyl]carbenium-chlorid (CI44045; Basic Blue No. 26), 2-[(4-(Ethyl(2-hydroxyethyl)amino)phenyl)azo]-6-methoxy-3-methylbenzothiazolium-methylsulfat (CI11154; Basic Blue No. 41), 8-Amino-2-brom-5-hydroxy-4-imino-6-[(3-(trimethylammonio)phenyl)amino]-1(4H)-naphthalinon-chlorid (CI56059; Basic Blue No. 99), Bis[4-(dimethylamino)-phenyl][4-(methylamino)phenyl]carbenium-chlorid (CI42535; Basic Violet No. 1), Tris(4-amino-3-methylphenyl)-carbenium-chlorid (CI42520; Basic Violet No. 2), Tris[4-(dimethylamino)phenyl]carbenium-chlorid (CI42555; Basic Violet No. 3), 2-[3,6-(Diethylamino)dibenzopyranium-9-yl]-benzoesäure-chlorid (CI45170; Basic Violet No. 10), Di(4-aminophenyl)(4-amino-3-methylphenyl)carbenium-chlorid (CI42510; Basic Violet No. 14), 1,3-Bis[(2,4-diamino-5-methylphenyl)azo]-3-methylbenzol (CI21010;Basic Brown No. 4), 1-[(4-Aminophenyl)azo]-7-(trimethylammonio)-2-naphtholchlorid (CI12250; Basic Brown No. 16), 1-[(4-Amino-2-nitrophenyl)azo]-7-(trimethylammonio)-2-naphthol-chlorid (CI12251; Basic Brown No. 17), 1-[(4-Amino-3-nitrophenyl)azo]-7-(trimethylammonio)-2-naphthol-chlorid (CI12251; Basic Brown No. 17), 3,7-Diamino-2,8-dimethyl-5-phenylphenazinium-chlorid (CI50240; Basic Red No. 2), 1,4-Dimethyl-5-[(4-(dimethylamino)phenyl)azo]-1,2,4-triazolium-chlorid (CI11055; Basic Red No. 22), 2-Hydroxy-1-[(2-methoxyphenyl)azo]-7-(trimethylammonio)-naphthalin-chlorid (CI12245; Basic Red No. 76), 2-[2-((2,4-Dimethoxyphenyl)amino)ethenyl]-1,3,3-trimethyl-3H-indol-1-ium-chlorid (CI48055; Basic Yellow No. 11), 3-Methyl-1-phenyl-4-[(3-(trimethylammonio)phenyl)-azo]-pyrazol-5-on-chlorid (CI12719; Basic Yellow No. 57), Bis[4-(diethylamino)phenyl]phenylcarbenium-hydrogensulfat(1:1) (CI42040; Basic Green No. 1).

Zum besseren Farbausgleich und zur Erzeugung von speziellen Nuancen besonders bewährt haben sich nichtionische Farbstoffe aus der Gruppe 1-Amino-2-[(2-hydroxyethyl)amino]-5-nitrobenzol (HC Yellow No. 5), 1-(2-Hydroxyethoxy)-2-[(2-hydroxyethyl)amino]-5-nitrobenzol (HC Yellow No. 4), 1-((2-Hydroxyethyl)amino]-2-nitrobenzol (HC Yellow No. 2), 2-[(2-Hydroxyethyl)amino]-1-methoxy-5-nitrobenzol, 2-Amino-3-nitrophenol, 1-(2-Hydroxyethoxy)-3-methylamino-4-nitrobenzol, 2,3-(Dihydroxypropoxy)-3-methylamino-4-nitrobenzol, 2-[(2-Hydroxyethyl)amino]-5-nitrophenol (HC Yellow No. 11), 3-[(2-Aminoethyl)amino]-1-methoxy-4-nitrobenzol-hydrochlorid (HC Yellow No.9), 1-[(2-Ureidoethyl)amino]-4-nitrobenzol, 4-[(2,3-Dihydroxypropyl)amino]-3-nitro-1-trifluormethyl-benzol (HC Yellow No. 6), 1-Chlor-2,4-bis-[(2-hydroxyethyl)amino]-5-nitrobenzol (HC Yellow No. 10), 4-[(2-Hydroxyethyl)amino]-3-nitro-1-methylbenzol, 1-Chlor-4-[(2-hydroxyethyl)amino]-3-nitrobenzol (HC Yellow No. 12), 4-[(2-Hydroxyethyl)amino]-3-nitro-1-trifluormethyl-benzol (HC Yellow No. 13), 4-[(2-Hydroxyethyl)amino]-3-nitro-benzonitril (HC Yellow No. 14), 4-[(2-Hydroxyethyl)amino]-3-nitro-benzamid (HC Yellow No. 15), 1-Amino-4-[(2-hydroxyethyl)amino]-2-nitrobenzol (HC Red No. 7), 2-Amino-4,6-dinitro-phenol, 2-Ethylamino-4,6-dinitrophenol, 4-Amino-2-nitrodiphenylamin (HC Red No. 1), 1-Amino-4-[di(2-hydroxyethyl)amino]-2-nitrobenzol-hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3), 4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxypropoxy)-1-((2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino] -2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)amino]-benzoesäure, 2-Chlor-6-methylamino-4-nitrophenol 2-Chlor-6-[(2-hydroxyethyl)amino]-4-nitrophenol, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1,4-Bis[(2-hydroxyethyl)amino]-2-nitrobenzol, 1-(2-Hydroxyethyl)amino-2-nitro-4-[di(2-hydroxyethyl)amino]-benzol (HC Blue No. 2), 1-Amino-3-methyl-4-[(2-hydroxyethyl)amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxy-propyl)amino]-4-[methyl-(2-hydroxyethyl)-amino]-2-nitrobenzol (HC Blue No. 10), 1-[(2,3-Dihydroxypropyl)amino]-4-[ethyl-(2-hydroxyethyl)amino]-2-nitrobenzol-hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxypropyl)amino]-2-nitrobenzol (HC Blue No. 6), 2-((4-Amino-2-nitrophenyl)amino)-5-dimethylamino-benzoesäure (HC Blue No. 13), 1,4-Di[(2,3-dihydroxypropyl)amino]-9,10-anthrachinon, 1-[(2-Hydroxyethyl)amino]-4-methylamino-9,10-anthrachinon (CI61505, Disperse Blue No. 3), 2-[(2-Aminoethyl)amino]-9,10-anthrachinon (HC Orange No. 5), 1-Hydroxy-4-[(4-methyl-2-sulfophenyl)amino]-9,10-anthrachinon, 1-[(3-Aminopropyl)amino]-4-methylamino-9,10-anthrachinon (HC Blue No. 8), 1-[(3-Aminopropyl)amino]-9,10-anthrachinon (HC Red No. 8), 1,4-Diamino-2-methoxy-9,10-anthrachinon (CI62015, Disperse Red No. 11, Solvent Violet No. 26), 1,4-Dihydroxy-5,8-bis[(2-hydroxyethyl)amino]-9,10-anthrachinon (CI62500, Disperse Blue No. 7, Solvent Blue No. 69), 1-[Di(2-hydroxyethyl)amino]-3-methyl-4-[(4-nitrophenyl)azo]-benzol (CI11210, Disperse Red No. 17), 4-[(4-Aminophenyl)azo]-1-[di(2-hydroxyethyl)amino]-3-methylbenzol (HC Yellow No. 7), 2,6-Diamino-3-[(pyridin-3-yl)azo]-pyridin, 2-((4-(Acetylamino)phenyl)-azo)-4-methylphenol (CI11855; Disperse Yellow No. 3).

Aus der Gruppe der direktziehenden Farbstoffe besonders zu erwähnen sind 2-Amino-4,6-dinitrophenol, 2-Ethylamino-4,6-dinitrophenol, 2-[(2-Hydroxyethyl)amino]-4,6-dinitrophenol und Farbstoffe der allgemeinen Formel (II), 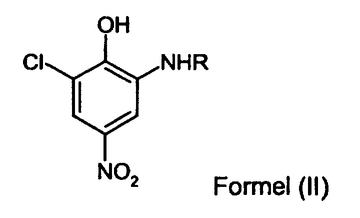 worin R Wasserstoff, Methyl, Ethyl oder Hydroxyethyl bedeutet.

Die vorstehend beschriebenen erfindungsgemäßen Kombinationen der Verbindungen der Formel (I) mit oxidativen Haarfarbvorstufen und/oder direktziehenden Farbstoffen werden zur Färbung in einer geeigneten Farbträgermasse appliziert.

Der Gegenstand der vorliegenden Erfindung betrifft daher auch ein Mittel zur oxidativen Färbung von Haaren, das durch Vermischen einer Farbträgermasse mit einem Oxidationsmittel unmittelbar vor der Anwendung hergestellt wird und dadurch gekennzeichnet ist, dass es mindestens eine Verbindung der Formel (I) sowie gegebenenfalls weitere Farbstoffvorstufen und/oder direktziehende Farbstoffe enthält.

In dem erfindungsgemäßen Mittel sind die Verbindungen der Formel (I) sowie die Farbvorstufen in einer Gesamtkonzentration von etwa 0,01 bis 10 Gewichtsprozent, vorzugsweise 0,2 und 6 Gewichtsprozent, enthalten. Die Gesamtkonzentration an direktziehenden Farbstoffen beträgt etwa 0,1 bis 10 Gewichtsprozent, vorzugsweise 0,1 bis 5 Gewichtsprozent.

Darüber hinaus können in der Farbträgermasse noch Antioxidantien, Parfümöle, Komplexbildner, Netzmittel, Emulgatoren, Penetrationsmittel, Puffersysteme, Konservierungsstoffe, Verdicker, Pflegestoffe und andere kosmetische Zusätze vorhanden sein.

Die Zubereitungsform für die Farbträgermasse sowie für das gebrauchsfertige Oxidationshaarfärbemittel kann beispielsweise eine Lösung, insbesondere eine wäßrige oder wäßrig-alkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze für Lösungen, Cremes, Emulsionen oder Gele sind zum Beispiel Lösungsmittel wie Wasser, niedere aliphatische Alkohole, beispielsweise Ethanol, n-Propanol und Isopropanol oder Glykole wie Glycerin und 1,2-Propylenglykol, weiterhin Netzmittel oder Emulgatoren aus den Klassen der anionischen, kationischen, amphoteren oder nichtionischen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, femer Verdicker wie höhere Fettalkohole, Stärke oder Cellulosederivate, weiterhin Vaseline, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet. Bezogen auf die Farbträgermasse sind dies zum Beispiel Netzmittel und Emulgatoren in Konzentrationen von etwa 0,5 bis 30 Gewichtsprozent, Verdicker in einer Menge von etwa 0,1 bis 25 Gewichtsprozent und die Pflegestoffe in einer Konzentration von etwa 0,1 bis 5,0 Gewichtsprozent.

Das gebrauchsfertige erfindungsgemäße Haarfärbemittel wird durch Mischen der Farbträgermasse mit einem flüssigen Oxidationsmittel unmittelbar vor der Anwendung hergestellt.

Als Oxidationsmittel kommen hauptsächlich Wasserstoffperoxid oder dessen Additionsverbindungen an Harnstoff, Melamin oder Natriumbromat in Form einer 1 bis 12prozentigen, vorzugsweise 6prozentigen, wässrigen Lösung in Betracht, wobei Wasserstoffperoxid besonders bevorzugt wird.

Die Farbträgermasse und das Oxidationsmittel werden hierbei im Gewichtsverhältnis von 5 zu 1 bis 1 zu 3 miteinander vermischt, wobei ein Gewichtsverhältnis von 1 bis 1 bis 1 zu 2 besonders bevorzugt ist.

Des pH-Wert des gebrauchsfertigen erfindungsgemäßen Haarfärbemittel stellt sich bei der Mischung der vorzugsweise alkalisch eingestellten Farbträgermasse mit dem meist sauer eingestellten Oxidationsmittel auf einen pH-Wert ein, der durch die Alkalimengen in der Farbträgermasse und die Säuremengen im Oxidationsmittel sowie durch das Mischungsverhältnis bestimmt wird. Der pH-Wert des gebrauchsfertigen Haarfärbemittels beträgt etwa 3 bis 11, wobei ein pH-Wert von 6 bis 10,5 bevorzugt ist.

Für die Einstellung des jeweiligen pH-Wertes der Farbträgermasse und des Oxidationsmittels können je nach dem gewünschten pH-Wert verdünnte organische oder anorganische Säuren, wie zum Beispiel Phosphorsäure, Ascorbinsäure und Milchsäure, oder Alkalien wie Monoethanolamin, Triethanolamin, 2-Amino-2-methyl-1-propanol, Ammoniak, Natronlauge, Kalilauge oder Tris(hydroxymethyl)aminomethan, verwendet werden.

Nach der Vermischung der vorstehend beschriebenen Farbträgermasse mit dem Oxidationsmittel wird eine für die Haarfärbebehandlung ausreichende Menge, je nach Haarfülle im allgemeinen etwa 60 bis 200 Gramm des erhaltenen gebrauchsfertigen Oxidationshaarfärbemittels auf das Haar aufgetragen.

Man läßt das erfindungsgemäße Haarfärbemittel etwa 10 bis 45 Minuten bei 15 bis 50 Grad Celsius, vorzugsweise 30 Minuten bei 40 Grad Celsius, auf das Haar einwirken und spült dann das Haar mit Wasser aus.

Gegebenenfalls wird das Haar im Anschluß an diese Spülung mit einem Shampoo gewaschen und eventuell mit einer verdünnten schwachen organischen Säure, wie zum Beispiel Zitronensäure oder Weinsäure nachgespült. Abschließend wird das Haar getrocknet.

Die nachfolgenden Beispiele sollen den Gegenstand näher erläutern, ohne ihn jedoch auf diese Beispiele zu beschränken.

### Beispiele

### Beispiel 1: Oxidationshaarfärbemittel, Creme basisch

| | |
|---|---|
| 15,00 g | Cetylstearylalkohol (50/50) |
| 5,00 g | Glycerinmonostearat |
| 2,00 g | Cocamide DEA |
| 10,00 g | Natriumlaurylethersulfat, 28%ige wässrige Lösung |
| 0,30 g | Ascorbinsäure |
| 0,40 g | Natriumsulfit |
| 4,50 g | Ammoniak, 25% ige wässrige Lösung |
| 0,56 g | 2-Aminomethyl-1,4-diamino-benzol-trihydrochlorid |
| X g | Kupplersubstanz gemäß Tabelle 1 |
| ad 100,00 g | Wasser, entmineralisiert |

Der pH-Wert der Cremes liegt zwischen 10 und 10,5.

Unmittelbar vor der Anwendung werden 100 Gramm der vorstehenden Farbträgermasse mit 100 Gramm einer 6%igen wäßrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40°C wird das Haar mit einem Shamopoo gewaschen, mit Wasser gespült und getrocknet. Die erhaltenen Farbnuancen und Farbintensitäten sind in Tabelle 1 zusammengefaßt.

**Tabelle 1**

| **Beispiel** | | **Kupplersubstanz** | **Farbton** | **Intensität** |
|---|---|---|---|---|
| **1a** | 0,28 g | Resorcin | mittelblond | (++) |
| **1b** | 0,27 g | 3-Aminophenol | aubergine | (o) |
| **1c** | 0,31 g | 5-Amino-2-methylphenol | rotviolett | (++) |
| **1d** | 0,64 g | 5-((2-Hydroxyethyl)amino)-2-methoxyanilindihydrochlorid | blauschwarz | (++) |
| **1e** | 0 ,47 g | 6-Amino-3,4-dihydro-2H-1,4-benzoxazinhydrochlorid | blau | (++) |
| **1f** | 0,59 g | 1,3-Diamino-4-methoxybenzol-sulfat | blau | (++) |
| **1g** | 0,36 g | 1-Chlor-2,4-dihydroxybenzol | gold | (++) |
| **1h** | 0,57 g | 3-Amino-6-methoxy-2-(methylamino)-pyridinhydrochlorid | aubergine | (++) |
| **1i** | 0,35 g | 5-Hydroxy-1,3-benzodioxol | kastanie | (++) |
| **1j** | 0,43 g | 5-Amino-1,3-benzodioxolhydrochlorid | dunkelbraun | (++) |
| **1k** | 0,44 g | 4-Methoxy-1-naphthol | blauviolett | (o) |
| **1l** | 0,61 g | 3,5-Diamino-2,6-dimethoxypyridindihydrochlorid | blauschwarz | (++) |
| **1m** | 0,40 g | 1,7-Dihydroxynaphthalin | blauviolett | (o) |
| **1n** | 0,31 g | 1,3-Dihydroxy-2-methylbenzol | rehbraun | (+) |
| **1o** | 0,45 g | 3-Dimethylaminophenylhamstoff | türkis | (++) |
| **1p** | 0,33 g | 4-Hydroxyindol | rotviolett | (++) |
| **1q** | 0,39 g | 3-Amino-2-chlor-6-methylphenol | violett | (++) |
| **1r** | 0,56 g | 2-Chlor-5-((2,2,2-trifluorethyl)amino)-phenol | graublau | (+) |
| **1s** | 0,45 g | 3-Amino-2,4-dichlorphenol | blauschwarz | (++) |
| **1t** | 0,36 g | 5-Amino-2-chlor-phenol | rotbraun | (+) |
| (o) = mittel, (+) = stark, (++) = sehr stark | | | | |

### Beispiel 2: Oxidationshaarfärbemittel, Creme basisch

| | |
|---|---|
| 15,00 g | Cetylstearylalkohol (50/50) |
| 5,00 g | Glycerinmonostearat |
| 2,00 g | Cocamide DEA |
| 10,00 g | Natriumlaurylethersulfat, 28%ige wässrige Lösung |
| 0,30 g | Ascorbinsäure |
| 0,30 g | Natriumsulfit |
| 5,15 g | Ammoniak, 25%ige wässrige Lösung |
| 0,10 g | 5-((2-Hydroxyethyl)amino)-1,3-benzodioxol-hydrochlorid |
| 1,65 g | 2-Aminomethyl-1,4-diamino-benzol-disulfat |
| 0,26 g | 2-Methylresorcin |
| 0,29 g | 2-Amino-6-chlor-4-nitro-phenol-hydrochlorid |
| 0,70 g | 5-Amino-2-methyl-phenol |
| 0,33 g | 1,4-Diamino-2-(2-hydroxyethyl)-benzol-sulfat |
| 0,43 g | 1,4-Diamino-2-methyl-benzol-sulfat |
| ad 100,00 g | Wasser, entmineralisiert |

Der pH-Wert der Creme beträgt 10,2.

Unmittelbar vor der Anwendung werden 100 Gramm der vorstehenden Farbträgermasse mit 100 Gramm einer 6%igen wässrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40°C wird das Haar mit einem Shampoo gewaschen, mit Wasser gespült und getrocknet. Man erhält eine braune Färbung mit roten Reflexen.

### Beispiel 3: Oxidationshaarfärbemittel, Creme sauer

| | |
|---|---|
| 15,00 g | Cetylstearylalkohol (50/50) |
| 5,00 g | Glycerinmonostearat |
| 2,00 g | Cocamid DEA |
| 10,00 g | Natriumlaurylethersulfat, 28%ige wässrige Lösung |
| 0,30 g | Ascorbinsäure |
| 0,40 g | Natriumsulfit |
| 0,56 g | 2-Aminomethyl-1,4-diamino-benzol-trihydrochlorid |
| X g | Kupplersubstanz gemäß Tabelle 2 |
| ad 100,00 g | Wasser, entmineralisiert |

Unmittelbar vor der Anwendung werden 100 Gramm der vorstehenden Farbträgermasse mit 100 Gramm einer 6%igen wässrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40°C wird das Haar mit einem Shampoo gewaschen, mit Wasser gespült und getrocknet. Die erhaltenen Farbnuancen und Farbintensitäten sowie der pH-Wert des gebrauchsfertigen Färbemittels sind in Tabelle 2 zusammengefaßt.

**Tabelle 2**

| **Beispiel** | | **Kupplersubstanz** | **pH-Wert** | **Farbton** | **Intensität** |
|---|---|---|---|---|---|
| **3a** | 0,28 g | Resorcin | 6,6 | braun | (+) |
| **3b** | 0,27 g | 3-Aminophenol | 5,8 | mittelblond | (o) |
| **3c** | 0,31 g | 5-Amino-2-methylphenol | 6,6 | rotviolett | (+) |
| **3d** | 0,64 g | 5-((2-Hydroxyethyl)amino)-2-methoxyanilindihydrochlorid | 6,7 | blau | (+) |
| (o) = mittel, (+) = stark, (++) = sehr stark | | | | | |

### Beispiel 4: Oxidationshaarfärbemittel in Gelform

| | |
|---|---|
| 15,00 g | Ölsäure |
| 3,00 g | Glycerin |
| 7,00 g | Isopropanol |
| 0,50 g | Ascorbinsäure |
| 0,40 g | Natriumsulfit |
| 0,40 g | Natriumhydroxid |
| 10,00 g | Ammoniak, 25%ige wässrige Lösung |
| 0,56 g | 2-Aminomethyl-1,4-diamino-benzol-trihydrochlorid |
| X g | Kupplersubstanz gemäß Tabelle 3 |
| ad 100,00 g | Wasser entmineralisiert |

Unmittelbar vor der Anwendung werden 100 Gramm der vorstehenden Farbträgermasse mit 100 Gramm einer 6%igen wässrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40°C wird das Haar mit einem Shampoo gewaschen, mit Wasser gespült und getrocknet. Die erhaltenen Farbnuancen und Farbintensitäten sowie der pH-Wert des gebrauchsfertigen Färbemittels sind in Tabelle 3 zusammengefaßt.

**Tabelle 3**

| **Beispiel** | | **Kupplersubstanz** | **pH-Wert** | **Farbton** | **Intensität** |
|---|---|---|---|---|---|
| **4a** | 0,28 g | Resorcin | 10,0 | mittelblond | (++) |
| **4b** | 0,27 g | 3-Aminophenol | 9,9 | palisander | (+) |
| **4c** | 0,31 g | 5-Amino-2-methylphenol | 10,2 | rotviolett | (+) |
| **4d** | 0,64 g | 5-((2-Hydroxy-ethyl)mino)-2-methoxyanilin-dihydrochlorid | 10,6 | blau | (++) |
| (o) = mittel, (+) = stark, (++) = sehr stark | | | | | |

### Beispiel 5: Oxidationshaarfärbemittel in Gelform

| | |
|---|---|
| 15,25 g | Ölsäure |
| 3,00 g | Glycerin |
| 8,00 g | Ethanol |
| 0,50 g | Ascorbinsäure |
| 0,40 g | Natriumsulfit |
| 10,30 g | Ammoniak, 25%ige wässrige Lösung |
| 0,35 g | 2-Aminomethyl-1,4-diamino-benzol-disulfat |
| 0,21 g | 1,4-Diamino-2-(2-hydroxyethyl)-benzol-sulfat |
| 0,50 g | 4,5-Diamino-1-(2-hydroxyethyl)-pyrazol-sulfat |
| 0,31 g | 3-Amino-phenol |
| 0,13 g | Resorcin |
| 0,12 g | 2-Chlor-6-(ethylamino)-4-nitro-phenol |
| ad 100,00 g | Wasser, entmineralisiert |

Unmittelbar vor der Anwendung werden 100 Gramm der vorstehenden Farbträgermasse mit 100 Gramm einer 6%igen wässrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40°C wird das Haar mit einem Farbpflegeshampoo gewaschen, mit Wasser gespült und getrocknet. Man erhält eine rotbraune Färbung mit hoher Sättigung und Brillanz.

### Beispiel 6: Haarfärbelösung mit einem basischen pH-Wert

| | |
|---|---|
| 10,00 g | Ethanol |
| 10,00 g | Natriumlaurylethersulfat, 28%ige wässrige Lösung |
| 10,00 g | Ammoniak, 25%ige wässrige Lösung |
| 0,30 g | Ascorbinsäure |
| 0,75 g | 2-Aminomethyl-1,4-diamino-benzol-trihydrochlorid |
| X g | Kupplersubstanz gemäß Tabelle 4 |
| ad 100,00 g | Wasser, entmineralisiert |

Unmittelbar vor der Anwendung werden 10 Gramm der vorstehenden Lösung mit 10 Gramm einer 6%igen wässrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40°C wird das Haar mit einem Shampoo gewaschen, mit Wasser gespült und getrocknet. Die erhaltenen Farbnuancen und Farbintensitäten sowie der pH-Wert des gebrauchsfertigen Färbemittels sind in Tabelle 4 zusammengefaßt.

**Tabelle 4**

| **Beispiel** | | **Kupplersubstanz** | **pH-Wert** | **Farbton** | **Intensität** |
|---|---|---|---|---|---|
| **6a** | 0,60 g | 1,3-Diamino-4-(2-hydroxy-ethoxy)-benzol-dihydrochlorid | 10,7 | blau | (+) |
| **6b** | 0,39 g | 3-Amino-2-chlor-6-methyl-phenol | 10,5 | violett | (+) |
| **6c** | 0,45 g | 3-Amino-2,4-dichlor-phenol | 10,6 | aubergine | (+) |
| **6d** | 0,56 g | 2-Chlor-5-((2,2,2-trifluorethyl)-amino)-phenol | 10,7 | blau | (+) |
| **6e** | 0,61 g | 3-Amino-6-methoxy-2-(methylamino)-pyridin-dihydrochlorid | 10,6 | blaugrün | (+) |
| **6f** | 0,36 g | 1-Naphthol | 10,9 | blauviolett | (+) |
| (o) = mittel, (+) = stark, (++) = sehr stark | | | | | |

### Beispiel 7: Haarfärbelösung mit einem basischen pH-Wert

| | |
|---|---|
| 10,00 g | Ethanol |
| 10,00 g | Natriumlaurylethersulfat, 28%ige wässrige Lösung |
| 10,00 g | Ammoniak, 25%ige wässrige Lösung |
| 0,30 g | Ascorbinsäure |
| 0,38 g | 2-Aminomethyl-1 ,4-diamino-benzol-trihydrochlorid |
| X g | Kupplersubstanz gemäß Tabelle 5 |
| ad 100,00 g | Wasser, entmineralisiert |

Unmittelbar vor der Anwendung werden 20 Gramm der vorstehenden Farbträgermasse mit 20 Gramm einer 6%igen wässrigen Wasserstoffperoxidlösung vermischt und das erhaltene gebrauchsfertige Oxidationshaarfärbemittel in der erforderlichen Menge auf gebleichte Haare aufgetragen. Nach einer Einwirkzeit von 30 Minuten bei 40°C wird das Haar mit einem Farbpflegehampoo gewaschen, mit Wasser gespült und getrocknet. Die erhaltenen Farbnuancen und Farbintensitäten sowie der pH-Wert des gebrauchsfertigen Färbemittels sind in Tabelle 5 zusammengefaßt.

**Tabelle 5**

| **Beispiel** | | **Kupplersubstanz** | **pH-Wert** | **Farbton** | **Intensität** |
|---|---|---|---|---|---|
| **7a** | 0,40 g | 1,3-Diamino-4-(2-hydroxy-ethoxy)-benzoldihydrochlorid | 10,5 | blau | (+) |
| **7b** | 0,26 g | 3-Amino-2-chlor-6-methyl-phenol | 10,5 | violett | (o) |
| **7c** | 0,30 g | 3-Amino-2,4-dichlorphenol | 10,6 | aubergine | (o) |
| **7d** | 0,37 g | 2-Chlor-5-((2,2,2-trifluorethyl)-amino)-phenol | 10,4 | blau | (+) |
| **7e** | 0,40 g | 3-Amino-6-methoxy-2-(methylamino)-pyridindihydrochlorid | 10,4 | blaugrün | (o) |
| **7f** | 0.24g | 1-Naphthol | 10.5 | blauviolett | (+) |
| (o) = mittel, (+) = stark, (++) = sehr stark | | | | | |

### Beispiel 8: Färbemittel

Eine gebrauchsfertige Creme nach Beispiel 1 wird auf die in der nachfolgenden Tabelle 6 angeführten Textilien aufgetragen. Nach einer Einwirkungszeit von 30 Minuten bei 40°C wird neutralisiert und mit Wasser gründlich ausgewaschen.

**Tabelle 6**

| **Beispiel** | **Fasermaterial** | **Färbemassegemäß Beispiel 1a** | **Färbemassegemäß Beispiel 1b** | **Färbemassegemäß Beispiel 1c** | **Färbemassegemäß Beispiel 1d** |
|---|---|---|---|---|---|
| **8a** | Baumwolle | mittelbraun (+) | hellbraun (+) | rosé (o) | blaugrau (+) |
| **8b** | Seide | mittelbraun (+) | braun (++) | rosé (++) | blau (+) |
| **8c** | Wolle | caramel (++) | dunkelbraun (++) | rotviolett (++) | blauschwarz (++) |
| **8d** | Nylon 66 | mittelblond (+) | hellbraun (+) | rotbraun (+) | aubergine (+) |
| (o) = mittel, (+) = stark, (++) = sehr stark | | | | | |

## Patentansprüche

1. Verwendung von 2-Aminomethyl-1,4-diamino-2-benzol oder dessen Salzen der Formel (I) in Färbemitteln für Keratinfasern, 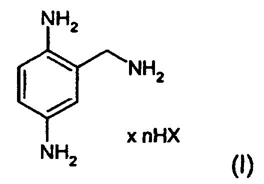 wobei n einen Wert von 0 bis 3 aufweist und HX für eine organische oder anorganische Säure steht.

2. Verwendung nach Anspruch 1, **dadurch gekennzeichnet, dass** die Säure ausgewählt ist aus Salzsäure und Schwefelsäure.

3. Mittel zur oxidativen Färbung von Haaren, welches durch Vermischen einer Farbträgermasse mit einem Oxidationsmittel unmittelbar vor der Anwendung hergestellt wird und **dadurch gekennzeichnet ist, dass** es mindestens eine Verbindung der Formel (I), 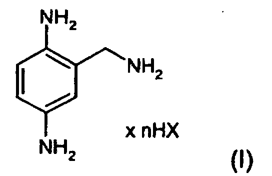 wobei n einen Wert von 0 bis 3 aufweist und HX für eine organische oder anorganische Säure steht, sowie gegebenenfalls weitere Farbstoffvorstufen und/oder direktziehende Farbstoffe enthält.

4. Mittel nach Anspruch 3, **dadurch gekennzeichnet, dass** es die Verbindungen der Formel (I) sowie die weiteren Farbstoffvorstufen in einer Gesamtmenge von 0,01 bis 10 Gewichtsprozent enthält.

5. Mittel nach Anspruch 3 oder 4, **dadurch gekennzeichnet, dass** die direktziehenden Farbstoffe in einer Gesamtmenge von 0,1 bis 10 Gewichtsprozent enthalten sind.

6. Mittel nach einem der Ansprüche 3 bis 5, **dadurch gekennzeichnet, dass** es unmittelbar vor der Anwendung durch Vermischen einer Farbträgermasse mit einem Oxidationsmittel in einem Gewichtsverhältnis von 5:1 bis 1:3 hergestellt wird.

## Claims

1. Use of 2-aminomethyl-1,4-diamino-2-benzene or salts thereof of the formula (I) in colorants for keratin fibres, 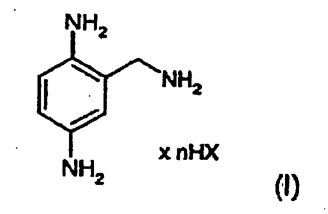 where n has a value from 0 to 3 and HX is an organic or inorganic acid.

2. Use according to Claim 1, **characterized in that** the acid is chosen from hydrochloric acid and sulphuric acid.

3. Composition for the oxidative colouring of hair which is prepared by mixing a colour carrier mass with an oxidizing agent directly prior to application and is **characterized in that** it comprises at least one compound of the formula (I), 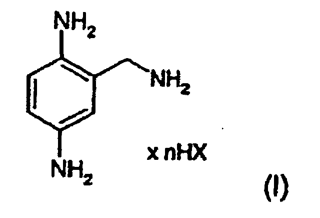 where n has a value from 0 to 3 and HX is an organic or inorganic acid, and optionally further dye precursors and/or direct dyes.

4. Composition according to Claim 3, **characterized in that** it comprises the compounds of the formula (I) and the further dye precursors in a total amount of from 0.01 to 10 percent by weight.

5. Composition according to Claim 3 or 4, **characterized in that** the direct dyes are present in a total amount of from 0.1 to 10 percent by weight.

6. Composition according to one of Claims 3 to 5, **characterized in that** it is prepared directly prior to application by mixing a colour carrier mass with an oxidizing agent in a weight ratio of from 5:1 to 1:3.

## Revendications

1. Utilisation du 2-aminométhyl-1,4-diamino-2-benzène ou de ses sels de formule (I) dans des produits colorants pour fibres de kératine, 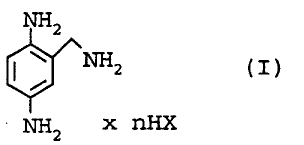 dans laquelle n a une valeur de 0 à 3 et HX représente un acide organique ou minéral.

2. Utilisation selon la revendication 1, **caractérisée en ce que** l'acide est choisi parmi l'acide chlorhydrique et l'acide sulfurique.

3. Produit pour la teinture des cheveux par oxydation, qui est préparé immédiatement avant l'emploi par prémélange d'une matière colorante avec un oxydant et est **caractérisé en ce qu'**il contient au moins un composé de formule (I) 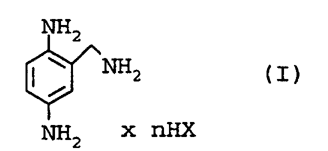 dans laquelle n a une valeur de 0 à 3 et HX représente un acide organique ou minéral, ainsi qu'éventuellement d'autres précurseurs de colorants et/ou des colorants directs.

4. Produit selon la revendication 3, **caractérisé en ce qu'**il contient les composés de formule (I) ainsi que les autres précurseurs de colorants en une quantité totale de 0,01 à 10 % en poids.

5. Produit selon la revendication 3 ou 4, **caractérisé en ce que** les colorants directs sont contenus en une quantité totale de 0,1 à 10 % en poids.

6. Produit selon l'une quelconque des revendications 3 à 5, **caractérisé en ce qu'**il est préparé immédiatement avant l'emploi, par mélange d'une matière colorante avec un oxydant en un rapport pondéral de 5:1 à 1:3.
